# EUROPÄISCHE PATENTSCHRIFT

(11) **EP 0 816 359 B1**
(45) Veröffentlichungstag und Bekanntmachung des Hinweises auf die Patenterteilung: **03.11.2004**
(21) Anmeldenummer: 97109548.4
(22) Anmeldetag: 12.06.1997
(51) Int. Cl.: C07D 403/04, A61K 31/505

(54) **4-Amino-2-ureido-pyrimidin-5-carbonsäureamide, Verfahren zu deren Herstellung, diese Verbindungen enthaltende Arzneimittel und deren Verwendung**
4-Amino-2-ureido-pyrimidine-5-carboxamides, processes for their preparation, medicaments containing these compounds, and their use
4-Amino-2-uréido-pyrimidine-5-carboxamides, procédés pour leur préparation, médicaments contenant ces composés, et leur application

(30) Priorität: 24.06.1996 DE 19625088
(43) Veröffentlichungstag der Anmeldung: 07.01.1998
(73) Patentinhaber: Aventis Pharma Deutschland GmbH, 65929 Frankfurt am Main (DE)
(72) Erfinder: Rackur, Gerhard, Dr., 65510 Idstein (DE); Böger, Hans Georg, Dr., 65529 Waldems Esch (DE); Krass, Norbert, Dr., 60487 Frankfurt (DE); Hoffmann, Axel, Dr., 65929 Frankfurt (DE); Leineweber, Michael, Dr., 65929 Frankfurt (DE)

(56) Entgegenhaltungen:
- EP-A- 0 206 297
- EP-A- 0 557 877
- EP-A- 0 557 879
- EP-A- 0 816 358
- DE-A- 2 853 220

## Beschreibung

Die Erfindung betrifft tertiäre Amide der 4-Amino-2-ureido-pyrimidin-5-carbonsäure sowie deren Säureadditionssalze.
Insbesondere betrifft die Erfindung substituierte 4-Amino-2-(imidazolin-2-on-1-yl)-pyrimidin-5-carbonsäure-[N-(fluoralkyl-N-(substituierte)phenyl-amide] und deren Säureadditionssalze.

Es ist bereits beschrieben worden, 4-Amino-2-ureido-pyrimidin-5-carbonsäure-(Nalkyl-N-phenyl-amide) zur Behandlung von Adipositas und von Lipidstoffwechselstörungen [vgl. Europ. Patentschrift 0 557 879] zu verwenden.

Die Stabilität im Metabolismus der als Arzneimittel vorgeschlagenen N-Phenylamide und zwar der alkylsubstituierten, tertiären Amide ist jedoch nicht ganz befriedigend. Eine hohe Stabilität im Metabolismus ist sehr wichtig, um Nebenwirkungen durch Metabolite möglichst auszuschließen.

Der Erfindung lag die Aufgabe zugrunde, Verbindungen zur Verfügung zu stellen, die eine hohe Stabilität im Metabolismus und eine therapeutisch verwertbare Wirkung bei Lipidstoffwechselstörungen, insbesonders eine hypolipidämische Wirkung aufweisen.

Überraschend wurde nun gefunden, daß tertiäre 4-Amino-2-ureido-pyrimidin-5-carbonsäure-(N- phenyl-amide), deren Amidstickstoffatom zweifach substituiert ist, also neben dem substituierten Phenylrest einen weiteren und zwar einen Fluoralkylrest trägt, eine gute lipidsenkende Wirkung entfalten, wobei die Verbindungen eine erhöhte Stabilität im Metabolismus gegenüber einer metabolischen Dealkylierung aufweisen.

Die Erfindung betrifft daher tertiäre 4-Amino-2-ureido-pyrimidin-5-carbonsäure-amide der Formel I, 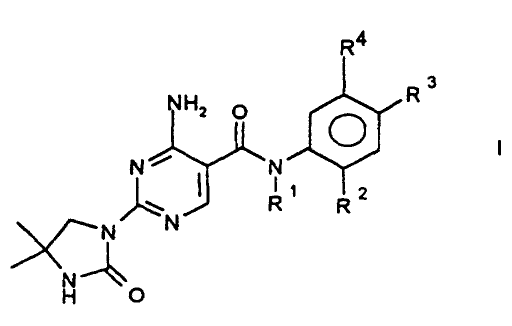 worin bedeuten
- R¹: 2,2,2-Trifluorethyl, 2,2,3,3,3-Pentafluorpropyl, 2,2,3,3,4,4,4-Heptafluorbutyl,
- R²: Fluor, Chlor, Wasserstoff, -CF₃, -OCF₃,
- R³: Fluor, Chlor, Wasserstoff, -CF₃, -OCF₃,
- R⁴: CF₃,
sowie deren physiologisch verträglichen Säureadditionssalze.

Unter physiologisch verträglichen Säureadditionssalzen werden in Wasser leicht lösliche, lösliche und wenig lösliche Verbindungen gemäß der Definition im "Deutschen Arzneibuch" (9. Ausgabe 1986, Amtliche Ausgabe, Deutscher Apotheker-Verlag Stuttgart), Seite 19 verstanden. Bevorzugt sind die Hydrochloride und Sulfate der Verbindungen.

Die Erfindung betrifft weiterhin 3 Verfahren zur Herstellung von 4-Amido-2-ureidopyrimidin-5-carbonsäureamiden der Formel I

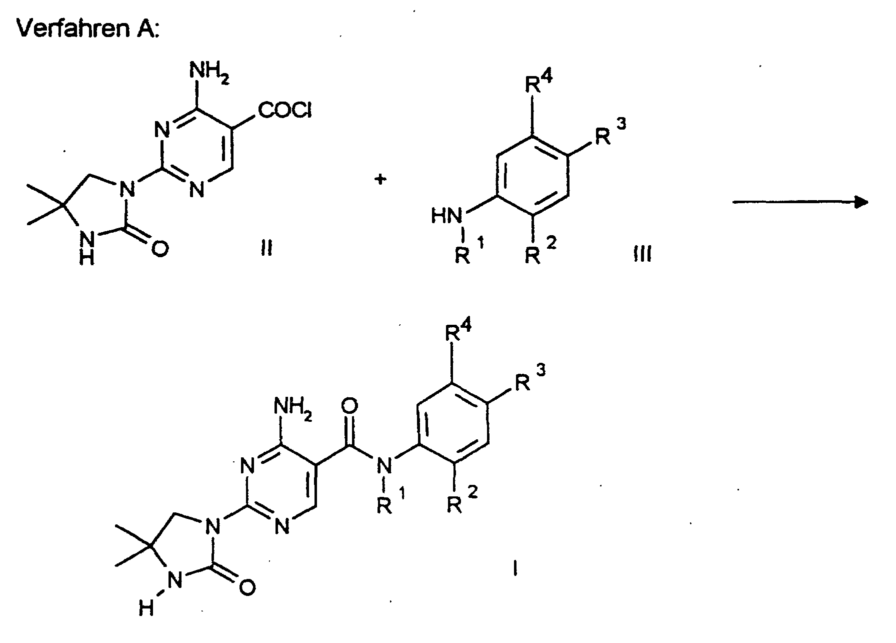

Verfahren A zur Herstellung der Verbindungen der Formel I, dadurch gekennzeichnet, daß man eine Verbindung der Formel II mit einer Verbindung der Formel III, in der R¹, R², R³ und R⁴ die zu Formel I angegebenen Bedeutungen haben, bei einer Temperatur von 0 °C bis 200 °C in einem geeigneten Lösungsmittel (wie z. B. DME) mit oder ohne Zusatz einer Hilfsbase (wie z. B. NEt₃) zu einer Verbindung der Formel I umsetzt, eine erhaltene Verbindung der Formel I gegebenenfalls in ein physiologisch verträgliches Salz überführt oder ein erhaltenes Salz gegebenenfalls in ein physiologisch verträgliches Salz überführt.

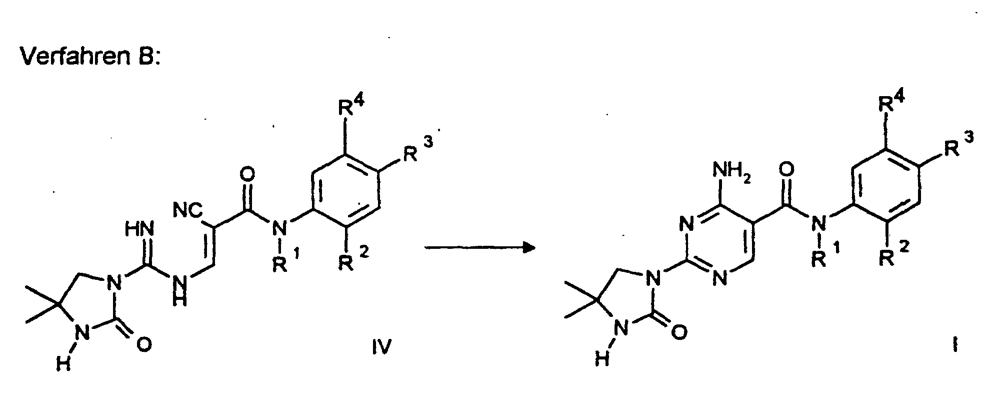

Verfahren B zur Herstellung der Verbindungen der Formel I, dadurch gekennzeichnet, daß man eine Verbindung der Formel IV, in der R¹, R², R³ und R⁴ die zu Formel I angegebenen Bedeutungen haben, zu einer Verbindung der Formel I cyclisiert. Die Herstellung der Verbindungen vom Typ IV ist, wie auch die Cyclisierung zu Verbindungen von Typ I, sind in der Europäischen Patentschrift 557 879 beschrieben.

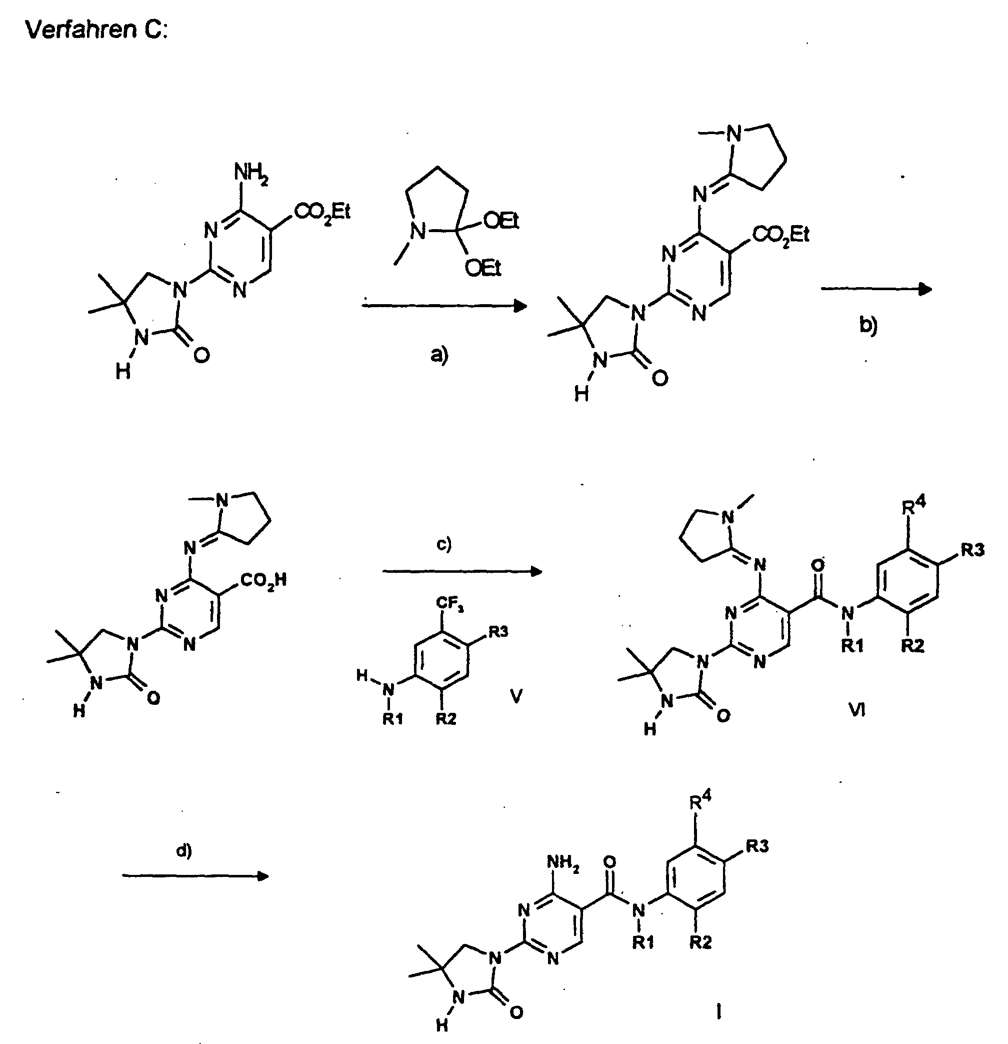

Verfahren C zur Herstellung der Verbindungen der Formel I, dadurch gekennzeichnet, daß man a) 4-Amino-2-(4,4-dimethyl-imidazolin-2-on-1-yl)-pyrimidin-carbonsäureethylester mit 2,2-Diethoxy-1-methyl-pyrrolidin in einem geeigneten Lösungsmittel, wie z. B. Ethanol bei einer Temperatur von 0 ° bis 150 °C zu 4-(1-Methyl-pyrrolidin-2-ylidenamino)-2-(4,4-dimethyl-imidazolin-2-on-1-yl)-pyrimidin-5-carbonsäureethylester umsetzt.

Der in der ersten Stufe erhaltene 4-(1-Methyl-pyrrolidin-2-ylidenamino)-2-(4,4-dimethyl-imidazolin-2-on-1-yl)-pyrimidin-5-carbonsäureethylester wird mit NaJ und TMSCI in einem geeigneten Lösungsmittel, wie z. B. Acetonitril, bei einer Temperatur von 0 ° bis 150 °C zu 4-(1-Methyl-pyrrolidin-2-ylidenamino)-2-(4,4-dimethyl-imidazolin-2-on-1-yl)-pyrimidin-5-carbonsäure umgesetzt (b).

Die in der zweiten Stufe erhaltene 4-(1-Methyl-pyrrolidin-2-ylidenamino)-2-(4,4-dimethyl-imidazolin-2-on-1-yl)-pyrimidin-5-carbonsäure wird mit einer Verbindung der Formel V umgesetzt, in der R¹, R², R³ und R⁴ die zu Formel I angegebenen Bedeutungen haben. Dies erfolgt in einem geeigneten Lösungsmittel, wie z. B. DMF bei einer Temperatur von 0 ° bis 150 °C in Gegenwart von TOTU und einer Hilfsbase, wie z. B. Triethylamin. Man erhält hierbei die Verbindung der Formel VI (c).

Die in der dritten Stufe erhaltene Verbindung der Formel VI, in der R¹, R², R³ und R⁴ die zu Formel I angegebenen Bedeutungen haben, wird in einem geeigneten Lösungsmittel, wie z. B. Isopropanol bei einer Temperatur von 0 ° - 150 °C in Gegenwart einer Hilfsbase, wie z. B. wäßrige Ammoniaklösung mit Ethylendiamin zu einer Verbindung der Formel I umgesetzt (d).

Die 4-Amino-2-(4,4-dimethyl-imidazolin-2-on-1yl)pyrimidin-5-carbonsäure, deren Säurechlorid das Ausgangsprodukt von Verfahren A bildet bzw. deren Ester das Ausgangsprodukt von Verfahren C bildet, wird wie folgt dargestellt:

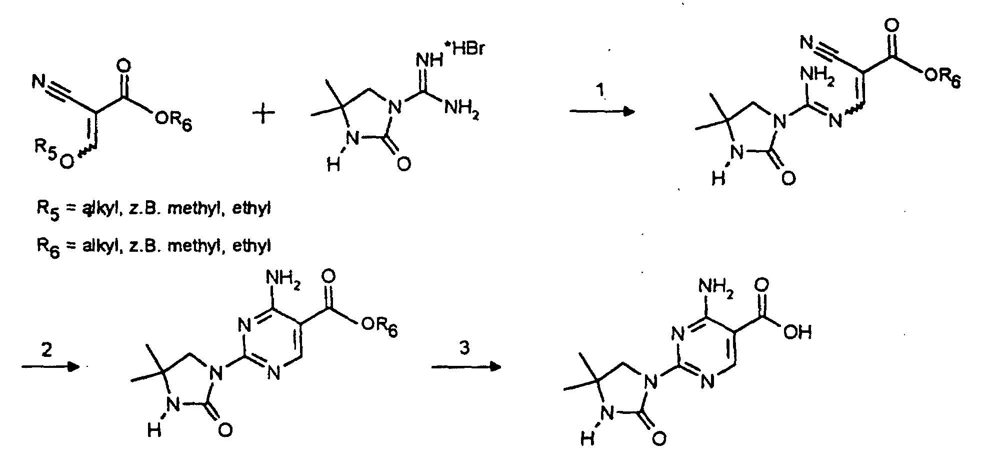

In der ersten Stufe werden 1-Amidino-4,4-dimethyl-imidazolin-2-on-hydrobromid und 2-Cyano-3-alkoxy-acrylsäurealkylester bei einer Temperatur von 0 ° bis 150 °C in einem geeigneten Lösungsmittel, wie z. B. Isopropanol in Gegenwart einer Base, wie z. B. KOH zu 3-(1-Amidino-4,4-dimethyl-imidazolin-2-on)-2-cyanoacrylsäurealkylester umgesetzt.

In der zweiten Stufe wird 3-(1-Amidino-4,4-dimethyl-imidazolin-2-on)-2-cyanoacrylsäurealkylester bei einer Temperatur von 0 ° bis 150 °C in einem geeigneten Lösungsmittel, wie z. B. Toluol in Gegenwart von Trifluoressigsäure oder Essigsäure zu 4-Amino-2-(4,4-dimethyl-imidazolin-2-on-1-yl)pyrimidin-5-carbonsäurealkylester cyclisiert.

In der dritten Stufe wird der 4-Amino-2-(4,4-dimethyl-imidazolin-2-on-1yl)pyrimidin-5-carbonsäurealkylester nach bekannten Methoden zur 4-Amino-2-(4,4-dimethylimidazolin-2-on-1-yl)-pyrimidin-5-carbonsäure verseift.

Die vorliegende Erfindung betrifft auch pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen inerten pharmazeutischen geeigneten Trägerstoffen sind pharmazeutisch unbedenkliche feste, halbfeste oder flüssige Verdünnungsmittel, Füllstoffe und Formulierungshilfsmittel jeder Art zu verstehen, die nach dem Vermischen mit dem Wirkstoff diesen in eine für die Verabreichung geeignete Form bringen.

Als geeignete Anwendungsformen der erfindungsgemäßen Verbindungen kommen beispielsweise Tabletten, Dragees, Kapseln, Pillen, wäßrige Lösungen, Suspensionen und Emulsionen, ggfs. sterile injizierbare Lösungen, nichtwäßrige Emulsionen, Suspensionen und Lösungen, Sprays sowie Zubereitungsformen mit protrahierter Wirkstoff-Freigabe in Betracht.

Die therapeutisch wirksamen Verbindungen sollen in den oben aufgeführten pharmazeutischen Zubereitungen zweckmäßig in einer Konzentration von etwa 0,1, 99,0, vorzugsweise von 0,5 bis bis 70,0 Gewichtsprozenten der Gesamtmischung vorhanden sein.

Die Anwendungskonzentrationen für Lösungen sowie Aerosole in Form von Spray betragen im allgemeinen 0,1 bis 20, vorzugsweise 0,5 - 5 Gewichtsprozent.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können oral, parenteral, intraperitoneal und/oder rectal appliziert werden.

Die z. B. als Hypolipidämika verwendbaren Verbindungen der vorliegenden Erfindung und ihre Salze können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Kapseln (Gelatinekapseln), welche den Wirkstoff zusammen mit Verdünnungsmitteln bzw. Trägerstoffen, z. B. Lactose, Dextrose, Rohrzucker, Mannitol, Sorbitol, Cellulose, verschiedenen Stärkearten und/oder Glycin, und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumcarbonat, Magnesiumaluminiumsilicat, Stärke, Gelatine, Traganath, Methylcellulose, Natriumcarboxymethylcellulose und/oder Polyvinylpyrrolidon und, falls benötigt, Farbstoffe, Geschmacksstoffe und Süßmittel. Injizierbare Lösungen sind vorzugsweise isotonische wässrige Lösungen oder Suspensionen, die sterilisiert sein können und Hilfsstoffe, wie Konservier-, Stabilisierungs-, Netzund/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die wenn erwünscht weitere pharmakologisch wirksame Stoffe enthalten können, werden z. B. mittels konventioneller Misch-Granulier- und Dragierverfahren, hergestellt und enthalten 0,1 % bis vorzugsweise 80 %, bevorzugt etwa 5 % bis etwa 65 %, des Wirkstoffs.

Die orale Anwendung erfolgt in pharmazeutisch üblichen Zubereitungen, beispielsweise in Form von Tabletten, Dragees oder Kapseln, die z. B. pro Tagesdosis 5 bis 1000 mg, vorzugsweise 20 bis 200 mg, des Wirkstoffes in Mischung mit einem gebräuchlichen Trägerstoff und/oder Konstituents enthalten, wobei Einzeldosen von 5 bis 200 mg, vorzugsweise ein- bis dreimal täglich, gegeben werden können.

Es kann jedoch erforderlich sein, von den genannten Dosierungen abzuweichen und zwar in Abhängigkeit von der Art und dem Körpergewicht des zu behandelnden Objekts, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die Verbindungen der Formel I und deren physiologisch verträglichen Salze stellen durch ihre Stabilität im Metabolismus ideale Arzneimittel zur Behandlung von Lipidstoffwechselstörungen, insbesonders von Hyperlipidämie dar. Die Verbindungen sind besonders geeignet durch Beeinflussung des LDL-Rezeptors, die Plasmaspiegel wirkungsvoll zu senken. Folgende Befunde belegen die pharmakologische Wirksamkeit der beschriebenen Verbindungen.
1a. In der allerseits als Modell anerkannten humanen Hepatozytom-Zellinie HepG2 werden die LDL-Rezeptor mRNA Spiegel durch die Verbindungen der Formel I erhöht (Tabelle I).
1b. Auch in Rattenlebern werden innerhalb weniger Stunden die LDL-Rezeptor mRNA Level durch Verbindungen der Formel gesteigert (Tabelle II).
Die Stimulierung liegt im Bereich von 170 bis 350 % der Kontrollen (Kontrolle = 100 %).
Die Präparation der mRNA wurde nach der Methode von Chomczynski, P. und Sacchi, N., Anal. Biochem. 162, 156 - 159 (1987) durchgeführt. Bei Organen (wie z. B. Leber) wurde das tiefgefrorene Gewebe auf Trockeneis zuvor im Mörser homogenisiert und die mRNA mittels Oligo dT nach Standardmethoden weiter angereichert (vgl. Sambrook, J., Fritsch, E. F. und Maniatis, T., Molecular Cloning, zweite Auflage, Cold Spring Harbor (1989); in dieser Methodensammlung finden sich auch Beschreibungen aller weiteren hier verwendeten relevanten molekularbiologischen Standardverfahren). 5 bis 20 µm der so gewonnenen gelösten mRNA wurden nach Standardverfahren denaturiert und auf 1 %igen horizontalen Agarosegelen aufgetrennt. Die mRNA wurde mittels Kapillarblot auf Hybond N Membranen (Amersham) transferiert. Als spezifische Hybridisierungsprobe diente ein partieller LDL-Rezeptor cDNA Klon und als interner Standard ein Plasmid, das ein β-Aktin Gen enthielt. Beide Plasmide wurden mittels eines random Primer Kit von Amersham bis zu einer spezifischen Aktivität von 5 x 10⁹ cpm/µg markiert. Vorhybridisierung, Hybridisierung und Waschen der Filter erfolgten nach Standardverfahren. Die Filter wurden anschließend auf Cronex 4 Filmen (Dupont) über Nacht bis zu 14 Tagen in Gegenwart einer Verstärkerfolie bei -70°C exponiert und die Hybridisierungssignale über die Filmschwärzungsintensität mit einem handelsüblichen Laserdensiometer quantifiziert. Anschließend wurde der Quotient aus der Intensität der LDL-Rezeptorbande und der Aktinbande als internem Standard zur Korrektur von Ausbeutevariationen bestimmt.
Tabelle I gibt die Stimulierung der LDL-Rezeptor-mRNA-Expression auf HepG2 Zellen durch ausgewählte Verbindungen der Formel I im Vollserum (Endkonzentration der Verbindungen 10⁻⁶ M) nach einer 16 h Inkubation wieder. Die HepG2 Zellen wurden in RPMI 1640 Standardmedium mit fötalem Kälberserum (Endkonzentration 10 %) inkubiert. Als Induktionskontrolle diente serumfreies RPMI Medium. Anschließend wurde die gesamt mRNA präpariert und mittels der Northern-Blot-Technik die relevanten LDL-Rezeptor-mRNA und β-Aktin-mRNA Level bestimmt. Der Quotient aus dem LDL-Rezeptor-mRNA Signal und dem β-Aktin-mRNA Signal der Kontrolle (ohne Substanzzugabe) wurde als 100 % gesetzt und die darüber unter dem Einfluß der Verbindungen erreichte Stimulierung der LDL-Rezeptor-mRNA Level in Prozent der Kontrolle ausgedrückt.

**Tabelle I**

| Verbindungen gemäß Beispiel | Konzentr. | LDL-Rezeptor-mRNA |
|---|---|---|
| 1 | 2x10⁻⁶ M | 220 |
| 10 | 2x10⁻⁶ M | 206 |
| 11 | 2x10⁻⁶ M | 290 |

Tabelle II zeigt die Stimulierung der LDL-Rezeptor-mRNA-Expression in Rattenlebem 6 Stunden nach einer Applikation ausgewählter Verbindungen der Formel I (Dosis von 30 mg/kg). Lebergewebe wurde entnommen und in flüssigem Stickstoff schockgefroren.
Anschließend wurde die mRNA wie beschrieben isoliert und mittels der Northern-Blot-Technik die relativen LDL-Rezeptor-mRNA-Level bestimmt.
mRNA Level von unbehandelten Kontrolltieren wurden als 100 % gesetzt und die Stimulierung der LDL-Rezeptor-mRNA in Prozent der Kontrolle errechnet.

**Tabelle II**

| Verbindungen gemäß Beispiel | Konzentr. | LDL-Rezeptor-mRNA |
|---|---|---|
| 1 | 30 mg/kg | 220 |
| 2 | 30 mg/kg | 245 |
| 3 | 30 mg/kg | 200 |
| 6 | 30 mg/kg | 216 |
| 8 | 30 mg/kg | 182 |
| 9 | 30 mg/kg | 193 |
| 14 | 30 mg/kg | 194 |
| 17 | 30 mg/kg | 195 |

### Versuche zur Stabilität im Metabolismus

Es ist bekannt, daß Verbindungen der Formel I mit R¹ = alkyl im Metabolismus zu Verbindungen der Formel I mit R¹ = H (im Folgenden Verbindung A genannt) abgebaut werden. Die folgenden Versuche dienen der Untersuchung der Dealkylierungstendenz der erfindungsgemäßen Verbindungen der Formel I im Vergleich zu 4-Amino-2-(4,4-dimethyl-2-oxo-imidazolin-1-yl)-pyrimidin-5-carbonsäure-(N-ethyl-N-3-trifluormethyl-phenyl)-amid)-hydrochlorid (Verbindung aus Beispiel 2 aus EP 0 557 879 - im folgenden Verbindung B genannt).

### 1.1. Human Hepatozyten

Die Human-Hepatozyten wurden von der Universität Pittsburgh, Abteilung Pathologie, bereitgestellt. Nach Perfusion der Leber wurde eine Zellsuspension mit William's E-Medium unter Zusatz von Insulin (7M), Dexamethason (7M), Penstrep und Fungazone (Gibco) hergestellt. Die Suspension wurde in Flaschen ausgestrichen, das Medium wurde mit 10% Kälberserum ergänzt. Nach dem Austausch des Mediums gegen serumfreies William's E wurden die Flaschen bei Raumtemperatur versandt. Nach der Ankunft wurde das Medium erneut ausgetauscht. Bei der Vitalprüfung mit Trypanblau zeigten die Zellen eine Vitalität von > 95%. Die Zeit zwischen der Leberperfusion und dem Beginn der Inkubationen betrug ca. 48 Stunden. Die Kulturen enthielten ca. 12 x 10⁶ Hepatozyten in 25 ml Kulturmedium.

### 1.2. Zubereitung von 9000 g-Leberfraktionen

### 1.2.1. Mensch

Bei -78°C gelagerte gefrorene Proben von Human-Leberstücken wurden aufgetaut, indem sie in eine 1,1%-ige Kaliumchlorid-Lösung bei einer Temperatur von 4°C eingegeben wurden. Ein solcher Gefrier/Auftau-Zyklus beeinträchtigt nicht die metabolisch relevanten Enzyme [P. J. Meier, H. K. Müller, B. Dick, U. A. Meyer; Gastroenterology 85, 682 (1983)]. Nach dem Auftauen wurden die Leberstücke nach Standardverfahren aufbereitet [A. Y. H. Lu, W. Levin; Biochem. Biophys. Res. Comm. 46, 1339 - 1344 (1972), P. G. Gervasi et al.; Xenobiotica 12/8, 517 (1982)]. Die Temperatur wurde während der Aufbereitung zwischen 0 und 4°C gehalten. Die 9000 g-Fraktionen von zehn Human-Lebern wurden gemischt, um interindividuelle Enzym-Unterschiede auszuschalten.

### 1.2.2. Eiweißgehalt von 9000 g-Leberfraktionen

Der Gehalt an Cytochrom P 450 der 9000 g-Fraktionen wurde wie folgt ermittelt [T. Omura, R. Sato; J. Biol. Chem. 239, 2370 (1964)]:

| Eiweißgehalt | [mg/ml] |
|---|---|
| Mensch* | 22 |

| | |
|---|---|
| *Gemisch aus Leberproben von 10 Personen | |

### 1.3. Inkubationsverfahren

### 1.3.1. Hepatozyten und 9000 g-Fraktionen

Die Testsubstanzen wurden in DMSO in einer Konzentration von ca. 10 mg/ml gelöst. Aliquote Teile dieser Lösung wurden unter sterilen Bedingungen zu den Hepatozytenkulturen bzw. den 9000 g-Fraktionen zugegeben. Die Inkubationen erfolgten bei 37°C in einer Atmosphäre mit 5 % CO₂ und > 90 % Luftfeuchtigkeit. Die Inkubationszeit betrug 3 Stunden für die 9000 g-Fraktionen bzw. 48 Stunden für die Hepatozyten. NADPH und Mg²⁺ wurden als Cofaktoren zu den 9000 g-Fraktionen zugegeben [P. G. Gervasi et al.; Xenobiotica 12/8, 517 (1982)]. Alle Proben wurden umgehend nach der Inkubation eingefroren und bis zur Analyse bei unter -20°C gelagert.

### 1.3.2. Kontroll- und Blindinkubationen

Die Testsubstanzen wurden im Puffersystem für die 9000 g-Fraktionen und das Hepatozyten-Kulturmedium in den entsprechenden Konzentrationen inkubiert, um die Stabilität der Substanz in den Kulturmedien nachzuweisen.

Alle Inkubationen mit Hepatozyten und 9000 g-Fraktionen wurden für jede Spezies und jeden Zeitpunkt ohne Zugabe einer Testsubstanz durchgeführt. Die erhaltenen Proben dienten als Kontrollen in der chromatographischen Analyse.

### 1.4. Bestimmung von A

Für die quantitative Bestimmung von A in den in vitro-Inkubationsgemischen wurde jeweils 0,25 µl jedes Gemischs mit 0,75 µl Rinderserum verdünnt. Diese Proben wurden nach dem folgenden Test zur Bestimmung von B freier Base und dem Metaboliten A freier Base in Serum analysiert:

Zu 0,1 ml Serum wurden 50 µl der Aufbereitungslösung eines internen Standards (10 µg einer Verbindung der Formel I mit R¹ = CH₃, R², R³ = H und R⁴ = CF₃ / ml Methanol), 0,1 ml Natriumacetatpuffer (0,4 M, pH-Wert 5,5) und 5 ml Ethylether zugegeben. Das Gemisch wurde 20 min. lang geschüttelt. Nach dem Zentrifugieren wurden 4 ml der organischen Phase übertragen, 3 ml n-Hexan zugegeben und mit 0,5 ml 1%-iger (V/V) wässriger Trifluoressigsäure extrahiert. Die obere organische Schicht wurde abgesaugt und verworfen. Der wässrige Rückstand wurde 30 min bei 40°C verdampft, um Reste des organischen Lösungsmittels zu entfernen. 100 µl der verbleibenden wässrigen Phase wurden in das HPLC-Gerät eingespritzt. Die HPLC-Analyse erfolgte auf einer C18 RP-Säule (TosoHaas Semi-Micro TSK gel ODS 80 TS) mit einer mobilen Phase aus 800 g Wasser, 270 g Acetonitril und 1 ml Trifluoressigsäure. Die Fließgeschwindigkeit betrug 0,2 ml/min. Die Mengenbestimmung der Analyten erfolgte durch Messen der Peakhöhen mithilfe eines UV-Detektors bei γ = 240 nm. Der Kalibrierungsbereich lag zwischen 50 und 0,05 µg/ml entsprechend 200 bis 0,2 µg/ml in der unverdünnten in vitro-Probe. Die Nachweisgrenze lag bei 0,05 µg/ml entsprechend 0,2 µg/ml in der unverdünnten in vitro-Probe.

Zusätzlich wurden nicht aufbereitete Proben sowohl mit Hilfe von Radio- als auch UV-Detektoren unter Verwendung des folgenden HPLC-Systems analysiert:
- Säule:: Nucleosil 100 oder 120 C 18,5 µm, 250 x 4[l.d.] mm (CTI GmbH, Idstein, Deutschland)

- Elutionsm. A:: 0,1 Gew.-% Ammoniumacetat in Wasser
- Elutionsm. B:: Elutionsmittel A / Acetonitril 1 : 4 (V/V)

| Gradient: | Zeit [min] | % A | % B | Flußgeschw. [ml/min] |
|---|---|---|---|---|
| | 0 | 100 | 0 | 1,0 |
| | 5 | 100 | 0 | 1,0 |
| | 35 | 75 | 25 | 1,0 |
| | 45 | 75 | 25 | 1,0 |
| | 46 | 0 | 100 | 1,9 |
| | 51 | 0 | 100 | 1,9 |
| | 52 | 100 | 0 | 1,0 |
| | 59 | 100 | 0 | 1,0 |

- Detektion: Radiodetector Ramona 92 (Raytest, Straubenhardt, Deutschland) UV bei 254 nm; Detektor-Modell 204 (Linear Instruments, Reno, Nevada, USA)

**Tabelle 1**

| Verbindung | Inkubationszeit [Std] | relativer Gehalt an Verbindung A |
|---|---|---|
| Verbindung B | 0,5 | 4 % |
| | 1 | 8 % |
| | 2 | 10 % |
| | 3 | 12 % |
| Beispiel 1 | 0,5 | < 2 % |
| | 1 | < 2% |
| | 2 | 3 % |
| | 3 | 4 % |
| Beispiel 2 | 0,5 | < 2 % |
| | 1 | < 2 % |
| | 2 | < 2 % |
| | 3 | < 2 % |
| Beispiel 12 | 0,5 | 2 % |
| | 1 | 4 % |
| | 2 | 6 % |
| | 3 | 9 % |

Aus Tabelle 1 ist abzulesen, daß die erfindungsgemäßen Verbindungen der Formel I eine größere Stabilität aufweisen als die Vergleichsverbindung B.

Die nachfolgenden Beispiele dienen zur näheren Erläuterung der Erfindung, ohne dieselbe auf in den Beispielen beschriebene Produkte und Ausführungsformen einzuschränken.

### Beispiel 1 (Verfahren A)

### 4-Amino-2-(4,4-dimethyl-imidazolin-2-on-1-yl)-pyrimidin-5-carbonsäure-N-(2,2,2-trifluor-ethyl)-N-[(3-trifluormethyl)phenyl]-amid Hydrochlorid

### 1. Stufe: Darstellung von 4-Amino-2-(4,4-dimethyl-imidazolin-2-on-1-yl)-pyrimidin-5-carbonsäure-N-(2,2,2-trifluorethyl)-N-[(3-trifluormethyl) phenyl]-amid

9,66 g 4-Amino-2-(4,4-dimethyl-imidazolin-2-on-1-yl)-5-pyrimidincarbonsäure [0,038 Mol] werden in 100 ml trockenem DME suspendiert, dann werden unter Rühren bei Raumtemperatur 11,5 ml [161 mmol] Thionylchlorid zugetropft und anschließend 5 Stunden am Rückfluß gekocht (85 ° - 90 °C). Zur Entfernung des überschüssigen Thionylchlorid werden 50 ml DME abdestilliert, jeweils (insgesamt 3 x wiederholen!) 50 ml frisches absolutes DME zugegeben und wieder 50 ml DME abdestilliert. Anschließend gibt man noch einmal 50 ml DME hinzu. Bei 40 °C wird dann eine Mischung von 11,2 g [0,046 Mol] N-(2,2,2-Trifluorethyl)-3-trifluormethylanilin und 5,75 g [0,046 Mol] Triäthylamin unter Rühren zu der Säurechloridsuspension hinzugetropft, wobei leichte Erwärmung eintritt. 5 Minuten wird bei 60 ° - 70 °C gerührt, dann werden weitere 2,3 ml Triäthylamin zugetropft und der Ansatz 30 Minuten bei 80 °C gerührt, anschließend bei Raumtemperatur über Nacht stehengelassen. Danach tropft man 150 ml H₂O hinzu (leichte Erwärmung) und zieht das DME am Rotationsverdampfer ab. Die saure Wasserphase wird einmal mit Essigester extrahiert, mit 2n wässriger NaOH-Lösung auf pH 8 - 9 gestellt und noch dreimal mit Essigester extrahiert. Die vereinigten Extrakte werden mit MgSO₄ getrocknet, filtriert und eingeengt. 4 g Rohprodukt fallen in schaumig-fester Form an. Die Reinigung erfolgt über Säulenchromatographie mit Kieselgel und Essigester/Methanol (10 : 1) als Laufmittel. Es werden 2,5 g gereinigtes Produkt als weiße Kristalle isoliert (13,8 % der Theorie, bezogen auf eingesetzte Carbonsäure). Smp.: 248 °C.
MS: m/e 477.2 (M⁺+1); 200 MHz ¹H-NMR (DMSO-d₆, ppm): 1.2(s, 6H), 3.55(s, 2H), 4.75(q, 2H), 7.0(brs, 2H), 7.28 (s, 1 H), 7.54-7.64 (m, 3H), 7.75-7.82 (m, 2H).

### 2. Stufe: Darstellung von 4-Amino-2-(4,4-dimethyl-imidazolin-2-on-1-yl)-pyrimidin-5-carbonsäure-N-(2,2,2-trifluorethyl)-N-[(3-trifluormethyl) phenyl]-amid Hydrochlorid

2,5 g (0,005 Mol) 4-Amino-2-(4,4-dimethyl-imidazolin-2-on-1-yl)-pyrimidin-5-carbonsäure-N-(2,2,2-trifluor-ethyl)-N-[(3-trifluormethyl)phenyl]amid werden in 60 ml Aceton aufgeschlämmt. Unter Rühren im Eisbad wird unter Feuchtigkeitsausschluß 1 ml einer bei 0 °C gesättigten ätherischen HCl-Lösung zugetropft. Es wird noch 2 Stunden im Kältebad nachgerührt und über Nacht bei Raumtemperatur stehengelassen. Danach werden 200 ml Äther zugegeben, nach beginnender Kristallisation auf 0 °C abgekühlt und die Kristalle abgesaugt. Ausbeute: 2,5 g weiße Kristalle (93% der Theorie). Smp.: > 300 °C.
200 MHz ¹H-NMR (DMSO-d₆, ppm): 1.25(s, 6H), 3.6(s, 2H), 4.75(t, 2H), 7.58-7.72 (m, 3H), 7.95(s, 1H), 8.04(s,1H), 8.62(s, 1H), 9.0(brs, 1H), 11,8-13.4(brs,1H).

### Beispiel 2 (Verfahren B)

### 4-Amino-2-(4,4-dimethyl-imidazolin-2-on-1-yl)-pyrimidin-5-carbonsäure-N-(2,2,3,3,4,4,4-heptafluorbutyl)-N-[(3-trifluormethyl)phenyl]-amid Hydrochlorid

### 1. Stufe: Darstellung von Cyanessigsäurechlorid

In einem 1-I-Vierhalskolben werden 30,6 g (1,375 Mol) Cyanessigsäure in 420 ml absolutem Ether gelöst und unter Eisbadkühlung werden portionsweise insgesamt 75 g PCl₅ (0,36 Mol) hinzugefügt. Anschließend wird bis zur vollständigen Auflösung des PCl₅ 3 Stunden bei Raumtemperatur nachgerührt, im Vakuum eingeengt und zweimal im Vakuum mit Toluol zur Entfernung des gebildeten POCl₃ abgeraucht. Das zurückbleibende rote Öl wird unverzüglich in die nächste Reaktion eingesetzt. (Darstellung nach Org. Synth. (1973), Coll. Vol. V, Seite 171-173.)

### 2. Stufe: Darstellung von N-(2,2,3,3,4,4,4-heptafluorbutyl)-N-[(3-trifluormethyl)phenyl)cyanacetamid

Das in der vorhergehenden Reaktion erhaltene Cyanessigsäurechlorid wird in 300 ml absolutem CH₂Cl₂ gelöst und in einem 1 I-Vierhalskolben vorgelegt. 58 g (0,17 Mol) N-(2,2,3,3,4,4,4-heptafluorbutyl)-3-trifluormethylanilin, gelöst in 150 ml absolutem CH₂Cl₂, und 23,5 ml (0,17 Mol) Triethylamin werden hinzugetropft, wobei die Reaktionsmischung zum Sieden kommt. Es wird noch 1 Stunde bei 40 °C gerührt und anschließend aufgearbeitet: Es werden 150 ml CH₂Cl₂ und 300 ml H₂O zugegeben und die Phasen getrennt. Die CH₂Cl₂-Phase wird 5x mit Wasser gewaschen, die H₂0-Phase 1x mit CH₂Cl₂ extrahiert. Die vereinigten CH₂Cl₂-Phasen werden mit MgSO₄ getrocknet, filtriert und im Vakuum eingeengt. Es bleiben 70 g eines braunen öligen Rückstandes übrig. Zur Reinigung wird eine Flash-Säulenchromatographie mit 500 g Kieselgel und n-Heptan/Essigester (1 :1) als Laufmittel durchgeführt. Die Chromatographie ergibt 48,6 g N-(2,2,3,3,4,4,4-heptafluorbutyl)-N-[(3-trifluormethyl)phenyl]cyanacetamid als dunkelgelben Schaum (~71 % der Theorie).
MS: m/e 411.1 (M⁺+1); 200 MHz ¹H-NMR (DMSO-d₆, ppm): 3.44(s, 2H), 4.68(t, 2H), 7.65-7.95(m, 4H).

### 3. Stufe: Darstellung von Ethoxymethylen-N-(2,2,3,3,4,4,4-heptafluorbutyl)-N-[(3-trifluormethyl)phenyl]cyanacetamid

In einem 250 ml-Vierhalskolben, versehen mit Kolonnenkopf und mechanischem Rührer mit Glashülse, werden bei Raumtemperatur 48,5 g (0,118 Mol) N-(2,2,3,3,4,4,4-heptafluorbutyl)-N-[(3-trifluormethyl)phenyl]cyanacetamid, 39,5 ml (0,237 Mol) Orthoameisensäure-triethylester und 44,9 ml (0,475 Mol) Essigsäureanhydrid zusammengegeben und im Ölbad langsam aufgeheizt. Bei einer Badtemperatur von 145 °C beginnt die Reaktion. Innerhalb von 1 - 2 Stunden werden ca. 60 ml abdestilliert. Nach dieser Zeit ist die Reaktion beendet (DC-Kontrolle). Im Vakuum bei einem Druck von 1 mm Hg und einer Badtemperatur von 140 °C werden alle flüchtigen Bestandteile abdestilliert. Der verbleibende Rückstand wird mit Essigester versetzt, am Rotationsverdampfer eingeengt und einmal mit Toluol abgedampft. Das verbleibende rotbraune Öl (56 g, ca. 100 % der Theorie) wird ohne weitere Reinigung in die nächste Stufe eingesetzt.
MS: m/e 467.1 (M⁺+1); 200 MHz H-NMR (DMSO-d₆, ppm): 1.22(t, 3H), 4.38(q, 2H), 4.78(t, 2H), 7.65-7.9 (m, 4H), 8.36(s, 1H).

### 4. Stufe: Darstellung von 4-Amino-2-(4,4-dimethyl-imidazolin-2-on-1-yl)-pyrimidin-5-carbonsäure-N-(2,2,3,3,4,4,4-heptafluorbutyl)-N-[(3-trifluormethyl) phenyl]amid

22,5 g (0,187 Mol) 1-Amidino-4,4-dimethyl-imidazolidin-2-on werden als Suspension in 300 ml absolutem DME unter Rühren und Feuchtigkeitsausschluß vorgelegt. Bei 0 °C werden 56 g (0,119 Mol) Ethoxymethylen-N-(2,2,3,3,4,4,4-heptafluorbutyl)-N-[(3-trifluormethyl)phenyl]-cyanacetamid in 300 ml absolutem DME langsam zugetropft. Nach Zugabe wird 2 Stunden bei Raumtemperatur gerührt. Nach dieser Zeit ist die Addition beendet (DC-Kontrolle). Zu dieser Lösung werden unter Rühren 125 ml Eisessig getropft und bei einer Temperatur von 50 °C 2 Stunden nachgerührt. Nach Stehenlassen über Nacht bei Raumtemperatur ist die Zyklisierung zum Pyrimidin-Derivat beendet. Zur Aufarbeitung wird das ausgefallene, als Nebenprodukt gebildete 5-Cyano-4-pyrimidon-Derivat abfiltriert. Das Filtrat wird eingeengt, der Eisessig mit Toluol abgedampft, und der halbfeste Rückstand in Essigester aufgenommen. Zusätzlich ausgefallene Mengen des gebildeten 5-Cyano-4-pyrimidon-Derivates werden wieder durch Filtration entfernt. Dann wird die Essigester-Phase 2 x mit 2n wässriger NaOH-Lösung und 2 x mit gesättigter Kochsalzlösung gewaschen, mit MgSO4 getrocknet, filtriert und am Rotationsverdampfer eingeengt, wonach als Rückstand 51 g Rohprodukt als dunkeles Öl verbleiben. Die Reinigung erfolgt über Säulenchromatographie mit Kieselgel und Essigester als Laufmittel. Nach Eindampfen des Lösungsmittels verbleiben 12,56 g 4-Amino-2-(4,4-dimethyl-imidazolin-2-on-1-yl)-pyrimidin-5-carbonsäure-N-(2,2,3,3,4,4,4-heptafluor-butyl)-N-[(3-trifluormethyl)phenyl]amid als gelbliche Kristalle (18 % der Theorie). Smp.: 188 °C.
MS: m/e 577.2 (M⁺+1); 200 MHz ¹H-NMR (DMSO-d₆, ppm): 1.2(s, 6H), 3.55(s, 2H), 4.8(t, 2H), 7.0(brs, 2H), 7.28 (s, 1H), 7.54-7.64 (m, 3H), 7.78-7.80 (m, 2H).

### 5. Stufe: Darstellung von 4-Amino-2-(4,4-dimethyl-imidazolin-2-on-1-yl)-pyrimidin-5-carbonsäure-N-(2,2,3,3,4,4,4-heptafluorbutyl)-N-[(3-trifluormethyl) phenyl]amid Hydrochlorid

12,5 g (0,023 Mol) 4-Amino-2-(4,4-dimethyl-imidazolin-2-on-1-yl)-pyrimidin-5-carbonsäure-N-(2,2,3,3,4,4,4-heptafluor-butyl)-N-[(3-trifluormethyl)phenyl]amid werden in 200 ml Aceton gelöst. Unter Rühren im Eisbad werden 6 ml einer bei 0 °C gesättigten ätherischen HCl-Lösung zugetropft. Es wird noch 2 Stunden unter Feuchtigkeitsausschluß im Kältebad nachgerührt und über Nacht bei Raumtemperatur stehengelassen. Danach werden 500 ml Äther zugegeben, nach beginnender Kristallisation auf 0 °C abgekühlt und die Kristalle abgesaugt. Ausbeute: 10 g leicht gelbliche Kristalle (71 % der Theorie). Smp.: 278 °C (Zers.). 200 MHz ¹H-NMR (DMSO-d₆, ppm): 1.25 (s, 6H), 3.60 (s, 2H), 4.80 (t, 2H), 7.58-7.75 (m, 3H), 7.95 (s, 1H), 8.04 (s, 1H), 8.64 (s, 1H), 9.02 (brs, 1H), 11,60-13.20 (brs, 1H).

### Beispiel 3:

### 4-Amino-2-(4,4-dimethyl-imidazolin-2-on-1-yl-pyrimidin-5-carbonsäure-N-(2,2,3,3,3-penta-fluorpropyl)-N-[(3-trifluormethyl)phenyl]-amid Hydrochlorid

Die Verbindung wird analog, Beispiel 1, Verfahren A hergestellt. Ausbeute 10 %; Smp.:> 300 °C; MS m/e = 527.3 (M⁺+1); 200 MHz ¹H-NMR (DMSO-d₆, ppm): 1.30 (s, 6H), 3.60 (s, 2H), 4.80 (t, 2H), 7.60-7.80 (m, 3H), 7.95 (s, 1 H), 8.05 (s,1 H), 8.60 (s, 1 H), 8.70 (brs, 1H), 9.10 (brs 1 H).

### Beispiel 4:

### 4-Amino-2-(4,4-dimethyl-imidazolin-2-on-1-yl)-pyrimidin-5-carbonsäure-N-(2,2,3,3,4,4,4-heptafluorbutyl)-N-[(3-trifluormethyl)phenyl]-amid Hydrochlorid

Die Verbindung wird analog Beispiel 1, Verfahren A hergestellt. Ausbeute 12 %; Smp.:278 °C (Zers.); MS m/e = 577.2 (M⁺+1); 200 MHz H-NMR (DMSO-d₆, ppm): 1.25 (s, 6H), 3.60 (s, 2H), 4.80 (t, 2H), 7.58-7.75 (m, 3H), 7.95 (s, 1 H), 8.04 (s, 1 H), 8.64 (s, 1 H); 9.02 (brs, 1 H), 11.60-13.20 (brs, 1 H).

### Beispiel 5:

### 4-Amino-2-(4,4-dimethyl-imidazolin-2-on-1-yl)-pyrimidin-5-carbonsäure-N-(2,2,2-trifluor-ethyl)-N-[(3-trifluormethoxy)phenyl]-amid

Die Verbindung wurde analog Beispiel 1, Verfahren A hergestellt. Ausbeute: 1 %. Smp.: 115 °C. MS: m/e 493.1 (M⁺ +1); 200 MHz ¹H-NMR (DMSO-d₆, ppm): 1.20 (s, 6H), 3.55 (s, 2H), 4.70 (t, 2H), 7.00 (brs, 2H), 7.20-7.40 (m, 4H), 7.40-7.50 (m, 1H), 7.80 (s, 1H).

### Beispiel 6:

### 4-Amino-2-(4,4-dimethyl-imidazolin-2-on-1-yl)-pyrimidin-5-carbonsäure-N-(2,2,2-trifluor-ethyl)-Nq(4-fluor-3-trifluormethyl)phenyl]-amid

Die Verbindung wurde analog Beispiel 1, Verfahren A hergestellt. Ausbeute: 1 %. Smp.: 90 °C. MS: m/e 495.2 (M⁺ +1); 200 MHz ¹H-NMR (DMSO-d₆, ppm): 1.30 (s, 6H), 3.60 (s, 2H), 4.70 (t, 2H), 7.00 (brs, 2H), 7.30 (s, 1H), 7.40-7.70 (m, 2H), 7.90 (m, 1H).

### Beispiel 7:

### 4-Amino-2-(4,4-dimethyl-imidazolin-2-on-1-yl)-pyrimidin-5-carbonsäure-N-(2,2,3,3,3-penta-fluorpropyl)-N-[(3-trifluormethoxy)-phenyl]-amid Hydrochlorid

Die Verbindung wurde analog Beispiel 2, Verfahren B hergestellt. Ausbeute: 11 %. Smp.: 287 °C. MS: m/e 543.1 (M⁺ +1); 200 MHz ¹H-NMR (DMSO, ppm): 1.25 (s, 6H), 3.60 (s, 2H), 4.80 (t, 2H), 7.35 (m, 1H), 7.50-7.60 (m, 3H), 8.00 (s, 1H), 8.50-9.20 (brs, 2H), 8.60 (s, 1 H).

### Beispiel 8:

### 4-Amino-2-(4,4-dimethyl-imidazolin-2-on-1-yl)-pyrimidin-5-carbonsäure-N-[(4-chlor-3-trifluormethyl)phenyl]-N-(2,2,2-trifluorethyl)-amid Hydrochlorid

Die Verbindung wurde analog Beispiel 1, Verfahren A hergestellt. Ausbeute: 12 %. Smp.: 268 - 270 °C; MS: m/e 511. 5 (M⁺+1); 200 MHz ¹H-NMR (DMSO-d₆, ppm): 1.20 (s, 6H), 3.55 (s, 2H), 4.75 (q, 2H), 7.05 (brs, 2H), 7.30 (s, 1 H), 7.55 (dd, 1 H), 7.70 (d, 1H), 7.90 (s, 1H), 7.95 (d,1 H).

### Beispiel 9:

### 4-Amino-2-(4,4-dimethyl-imidazolin-2-on-1-yl)-pyrimidin-5-carbonsäure-N-[(4-chlor-3-trifluormethyl)phenyl]-N-(2,2,3,3,3-pentafluorpropyl)-amid

Die Verbindung wurde analog Beispiel 2, Verfahren B hergestellt. Ausbeute: 12 %. Smp.: 232 - 234 °C; MS: m/e 561.8 (M⁺+1); 200 MHz ¹H-NMR (DMSO-d₆, ppm): 1.20 (s, 6H), 3.55 (s, 2H), 4.80 (t, 2H), 7.00 (brs, 2H), 7.30 (s, 1H), 7.55 (dd, 1H), 7.70 (d, 1H), 7.90 (s, 1 H), 7.95 (d, 1 H).

### Beispiel 10:

### 4-Amino-2-(4,4-dimethyl-imidazolin-2-on-1-yl)-pyrimidin-5-carbonsäure-N-[(4-chlor-3-trifluormethyl)phenyl]-N-(2,2,3,3,4,4,4-heptafluorbuyl)]-amid

Die Verbindung wurde analog Beispiel 2, Verfahren B hergestellt. Ausbeute: 10 %. Smp.: 228 - 230 °C; MS: m/e 611.6 (M⁺+1); 200 MHz ¹H-NMR (DMSO-d₆, ppm): 1.20 (s, 6H), 3.55 (s, 2H), 4.80 (t, 2H), 7.05 (brs, 2H), 7.30 (s, 1H), 7.55 (dd, 1H), 7.70 (d, 1 H), 7.90 (s, 1 H), 7.95 (d, 1 H).

### Beispiel 11:

### 4-Amino-2-(4,4-dimethyl-imidazolin-2-on-1-yl)-pyrimidin-5-carbonsäure-N-[(6-chlor-3-trifluormethyl)phenyl]-N-(2,2,3,3,3-pentafluorpropyl)-amid Hydrochlorid

Die Verbindung wurde analog Beispiel 1, Verfahren A hergestellt. Ausbeute: 29 %. Smp.: 152 °C; MS: m/e 561.5 (M⁺+1); 200 MHz ¹H-NMR (DMSO-d₆, ppm): 1.20 (s, 6H), 3.55 (s, 2H), 4.75 (t, 2H), 7.10 (brs, 2H), 7.35 (s, 1H), 7.70 (s, 2H), 7.75 (s, 1H), 8.30 (s, 1 H).

### Beispiel 12:

### 4-Amino-2-(4,4-dimethyl-imidazolin-2-on-1-yl)-pyrimidin-5-carbonsäure-[N-(3-trifluormethylphenyl)-N-(2-fluor-ethyl)]-amid

Die Verbindung wurde analog Beispiel 1, Verfahren A hergestellt. Ausbeute: 51 %. Smp.: 210 °C; MS: m/e 441 (M⁺+1); 200 MHz ¹H-NMR (DMSO, ppm): 1.30 (s, 6H), 3.75 (s, 2H), 4.15 (dt, 2H), 4.70 (dt, 2H), 6.40 (brs, 2H), 7.20-7.30 (m, 2H), 7.35-7.55 (m, 3H), 7.75 (s, 1H).

### Beispiel 13:

### 4-Amino-2-(4,4-dimethyl-imidazolidinyl-n-2-on-1-yl)-pyrimidin-5-carbonsäure-5-[N-(2,2,2-trifluorethyl)-N-(3-trifluormethyl-6-chlor-phenyl)]-pyrimidincarboxamid.

Die Verbindung wurde analog Beispiel 2, Verfahren B hergestellt. Ausbeute: 21 %. Smp.: 222 °C; MS: m/e 511. (M⁺+1); 200 MHz ¹H-NMR (DMSO-d₆, ppm): 1.22 (s, 6H), 3.60 (s, 2H), 4.46 und 4.70 (2 x brs, 2H), 7.82 (m, 2H), 7.94 (s, 1H), 8.23 (s, 1 H), 8.63 (s, 1 H), 8.70 (brs, 2H).

### Beispiel 14:

### 4-Amino-2-(4,4-dimethyl-imidazoli-n-2-on-1-yl)-pyrimidin-5-carbonsäure)-[N-(3-trifluormethyl-6-chlor-phenyl)-N-(2,2,3,3,4,4,4-heptafluorbutyl)]-amid.

Die Verbindung wurde analog Beispiel 2, Verfahren B hergestellt. Ausbeute: 9 %. Smp.: 203 °C; MS: m/e 611.6 (M⁺+1); 200 MHz ¹H-NMR (DMSO-d₆, ppm): 1.20 (s, 6H), 3.55 (s, 2H), 4.78 (t, 2H), 7.08 (brs, 2H), 7.33 (s, 1H), 7.73 (s, 2H), 7.78 (s, 1H), 8.34 (s, 1 H).

### Beispiel 15:

### 4-Amino-2-(4,4-dimethyl-imidazoli-n-2-on-1-yl)pyrimidin-5-carbonsäure-[N-(3-trifluormethylphenyl)-N-(2,2,3,3,4,4,5,5,5-nonafluorpentyl)]-amid.

Die Verbindung wurde analog Beispiel 2, Verfahren B hergestellt. Ausbeute: 15 %. Smp.: 174 - 176 °C; MS: m/e 627 (M⁺+1); 200 MHz ¹H-NMR (DMSO-d₆, ppm): 1.20 (s, 6H), 3.55 (s, 2H), 4.82 (t, 2H), 7.03 (brs, 2H), 7.28 (s, 1 H), 7.55-7.70 (m, 3H), 7.80 (s, 2H).

### Beispiel 16 (Verfahren C):

### 4-Amino-2-(4,4-dimethyl-imidazolin-2-on-1-yl-pyrimidin-5-carbonsäure)-[N-(3-trifluormethylphenyl)-N-(2,2,2-trifluorethyl)]amid

### 1. Stufe: Synthese von 4-(1-Methyl-pyrrolidin-2-ylidenamino)-2-(4,4-dimethyl-2-imidazolin-2-on-1-yl)pyrimidin-5-carbonsäureethylester

1 g = 3.58 mMol 4-Amino-2-(4,4-dimethyl-imidazolidin-2-on-1-yl)pyrimidin5-carbonsäureethylester werden in 20 ml Ethanol gelöst, mit 10 ml 2,2-Diethoxy-1-methylpyrrolidin versetzt und 1 h bei Raumtemperatur gerührt. Danach wird das Lösungsmittel und überschüssiges NMP-Diethylacetal am Rotationsverdampfer abdestilliert, der Rückstand mit Diethylether verrührt und abgesaugt. Der Rückstand wird in EtOH heiß gelöst, mit Aktivkohle aufgekocht, filtriert und eingeengt. Man erhält 1.12 g 4-(1-Methyl-pyrrolidin-2-ylidenamino)-2-(4,4-dimethyl-2-oxo-1-imidazolidin-2-on-1-yl)pyrimidin-5-carbonsäureethylester. Ausbeute: 86.9 %.
Schmelzpunkt: 169 °C
MS: m/e 361.2 (M⁺+1)
¹H NMR (200 MHz, DMSO-d₆, ppm), d [ppm]: 8.55 (s,1H), 7.35 (s,1H), 4.2 (q,2H), 3.73 (s,2H), 3.48 (t,2H), 3.03 (t,2H), 3.0 (s,3H), 1.98 (p,2H), 1.28 (t(verdeckt) + s, 9H)

### 2. Stufe: Synthese von 4-(1-Methyl-pyrrolidin-2-ylidenamino)-2-(4,4-dimethylimidazolidin-2-on-1-yl)pyrimidin-5-carbonsäure

2.1. 4 g = 11.1 mMol 4-(1-Methyl-pyrrolidin-2-ylidenamino)-2-(4,4-dimethylimidazolidin-2-on-1-yl)-pyrimidin-5-carbonsäureethylester werden mit 5.0 g = 33.3 mMol trockenem NaJ in 100 ml trockenem CH₃CN gelöst (Argon). Die Lösung wird zum Rückfluß erhitzt, und man tropft langsam 4.25 ml = 3.64 g = 33.3 mMol TMSCI zu. Die Reaktionsmischung wird 55 h unter Rückfluß gekocht. Danach wird ein weiteres Äquivalent NaJ und TMSCI zugegeben und weitere 15 h erhitzt. Die abgekühlte Reaktionsmischung wird abgesaugt und mit CH₃CN gewaschen. Der Rückstand wird in Wasser verrührt, erneut abgesaugt, mit wenig Ethanol und Diethylether gewaschen und im Vakuum getrocknet. Man erhält 2.52 g eines farblosen Pulvers.
   Ausbeute: 68,3 %,
   MS: m/e 333.1 (M⁺+1)
   Schmelzpunkt: 298 °C (Zers.)
   ¹H NMR (200 MHz, DMSO-d₆, ppm), d [ppm]: 14.4 (s,breit, 1H), 8.88 (s,1H), 8.15 (s,1H), 3.83 (t+s, 4H), 3.6 (t,2H), 3.23 (s,3H), 2.18 (p,2H), 1.33 (s,6H)
2.2. 1g = 3.98 mmol 4-Amino-2-(4,4-dimethyl-imidazolidin-2-on-1-yl)pyrimidin-5-carbonsäure werden in einer Mischung aus 20 ml trockenem Pyridin und 10 ml NMP-Diethylacetal suspendiert und 48 - 72 h bei Raumtemperatur gerührt. Man erhält bald eine rotbraune Lösung. Die Lösung wird mit ca. 50 ml Dichlormethan verdünnt, mit ca. 50 ml Wasser versetzt und dann unter Rühren mit Eisessig versetzt. Dabei scheidet sich an der Trennschicht zwischen organischer und wäßriger Phase ein gelblicher Niederschlag ab. Dieser Niederschlag wird abgesaugt, mit Wasser und dann mit Ethanol gewaschen und getrocknet. Man erhält 0,35 g eines farblosen Pulvers.
   Ausbeute: 26,5 %
   Schmelzpunkt: Zers. ab 295 °C
2.3. 5 g = 19.9 mMo l 4-Amino-2-(4,4-dimethyl-imidazolidin-2-on-1-yl) pyrimidin-5-carbonsäure werden unter Rühren in 50 ml trochenem Dichlormethan suspendiert. Es werden 10 ml NMP-Diethylacetal (2) zugegeben (leichte Erwärmung der Reaktionsmischung) und die Suspension wird 24 - 48 h bei Raumtemperatur gerührt. Die gelbliche Suspension wird abgesaugt und der Rückstand mit Dichlormethan gewaschen. Man erhält 2,07 g eines farblosen Pulvers.
   Ausbeute: 31,3 %
   Schmelzpunkt: ab 295 °C Zersetzung
   ¹H NMR 200 MHz, DMSO-d₆, ppm), d [ppm]: 14.4 (s,breit, 1 H), 8.88 (s,1H), 8.15 (s,1H), 3.83 (t+s, 4H), 3.6 (t,2H), 3.23 (s,3H), 2.18 (p,2H), 1.33 (s,6H)

### 3. Stufe: Synthese von 4-(1-Methyl-pyrrolidin-2-ylidenamino)-2-(4,4-dimethylimidazolidin-2-on-1-yl)pyrimidin-5-carbonsäure- [N-(2,2,2-trifluorethyl)]-N-3-trifluor-methylphenyl)]amid

1 g = 3 mMol 4-(1-Methyl-pyrrolidin-2-ylidenamino)-2-(4,4-dimethyl-imidazolidin-2-on-1-yl)pyrimidin-5-carbonsäure und 0,73 g = 3 mMol N-(2,2,2-Trifluorethyl)-3-trifluormethylanilin werden in 20 ml trockenem DMF bei 0 °C suspendiert und unter Rühren mit 0,99 g = 3 mMol TOTU (O-{[Cyano(ethoxycarbonyl)methyliden]-amino}-1,1,3,3-tetramethyluronium-tetrafluoroborat) und 0,416 ml NEt₃ = 3mMol versetzt. Man rührt 10' bei 0 °C, läßt auf Raumtemperatur erwärmen, gibt nach 1 h weitere 0,416 ml (=3mMol) NEt₃ nach und rührt die Reaktionsmischung anschließend über Nacht bei 100 °C Badtemperatur. Die Reaktionslösung wird eingeengt, der Rückstand mit Diethylether verrührt und abgesaugt. Der Rückstand wird mit gesättigter Natriumcarbonat-Lösung verrührt und die wäßrige Phase mehrmals mit Essigester extrahiert. Die Essigester-Phase wird eingeengt und der Rückstand über Kieselgel mit Essigester/Methanol 9/1 chromatographiert. Man erhält 0,47 g des Anilids als Öl.
Ausbeute: 28,4 %
MS: m/e 558 (M⁺ + 1)
¹H NMR (200 MHz, DMSO-d₆), d [ppm]: 8.22 (s,1H), 7.66 (s,1H), 7.59 - 7.45 (m,3), 4.8 q,2H), 3.58 (s,2H), 3.42 (t,2H), 3.0 (s,3H), 2.2 (t,2H), 1.91 (p,2H), 1.22 (s,6H)

### 4. Stufe: Synthese von 4-Amino-2-(4,4-dimethyl-imidazolidin-2-on-1-yl)-pyrimidin-5-carbonsäure-[N-(3-trifluormethylphenyl)-N-(2,2,2-trifluorethyl)]-amid

84 mg = 0.15 mMol 4-(1-Methyl-pyrrolidin-2-ylidenamino)-2-(4,4-dimethylimidazolidin-2-on-1-yl)pyrimidin-5-carbonsäure-N-[(2,2,2-trifluorethyl)]-N(3-trifluormethylphenyl)amid werden in einer Mischung aus 5 ml Isopropanol mit 3 ml konzentrierter wäßriger Ammoniaklösung suspendiert, mit 3 Tropfen Ethylendiamin versetzt und 24 - 48 h unter Rückfluß gekocht. Die Reaktionslösung wird eingeengt und über Kieselgel mit einer Mischung aus EE/MeOH 9/1 chromatographiert. Man erhält ein farbloses Öl, welches auf Zugabe von Diethylether im Eisbad kristallisiert. Man erhält 0,3 g 4-Amino-2-(4,4-dimethyl-imidazolidin-2-on-1-yl)-5-Pyrimidincarbonsäure[N-(3-trifluormethylphenyl)-N-(2,2,2-trifluorethyl)]-amid als farblose Kristalle.
Ausbeute: 44,7 %
Schmelzpunkt: 248 °C
H NMR (DMSO-d₆, ppm) : 1.2 (s,6H), 3.55 (s,2H), 4.75 (q,2H), 7.0 (s,breit,2H), 7.28 (s,1H), 7.64-7.54 (m,3H), 7.82-7.75 (m,2H)

### Beispiel 17:

4-Amino-2-(4,4-dimethyl-imidazolidin-2-on-1-yl)-5-pyrimidin-5-carbonsäure[N-(3-trifluormethyl)-4-fluorphenyl)-N-(2,2,3,3,4,4,4-heptafluorbutyl)]amid:
Die Verbindung wurde analog Beispiel 1, Verfahren A hergestellt. Ausbeute 5 %; Smp.: 128 °C (Zers.)
MS m/e = 595.3 (M⁺+1); 200 MHz ¹H-NMR (DMSO-d₆, ppm): 1.20 (s, 6H), 3.60 (s, 2H), 4.78 (t, 2H), 7.0 (brs, 2H), 7.20 (s, 1H), 7.42 - 7.95 (m, 3H).

### Beispiel 18:

### 4-Amino-2-(4,4-dimethyl-imidazolidin-2-on-1-yl)-pyrimidin-5-carbonsäure[N-(2-fluor-5-trifluormethyl-phenyl)-N-(2,2,2-trifluorethyl)]amid-hydrochlorid:

Die Verbindung wird analog Beispiel 2 hergestellt. Ausbeute 15 %; Smp.: 295 °C (Zers.); MS m/e = 495.1 (M⁺+1); 200 MHz ¹H-NMR (DMSO-d₆, ppm): 1.30 (s, 6H), 3.60 (s, 2H), 4.60 - 4.90 (brs, 2H), 7.55 (m, 1H), 7.85 (m, 1H), 7.95 (s, 1H), 8.25 (m, 1H), 8.60 (brs, 1H), 8.65 (s, 1H), 9.15 (brs, 1H).

### Beispiel 19:

### 4-Amino-2-(4,4-dimethyl-imidazolidin-2-on-1-yl)-pyrimidin-5-carbonsäure[N-(2-fluor-5-trifluormethyl-phenyl)-N-(2,2,3,3-3-pentafluoropropyl)]amid:

Die Verbindung wird analog Beispiel 2 hergestellt. Ausbeute 10 %; Smp.: 240 °C (Zers.); MS m/e = 545.2 (M⁺+1); 200 MHz ¹H-NMR (DMSO-d₆, ppm): 1.30 (s, 6H), 3.60 (s, 2H), 4.80 (t, 2H), 7.00 (brs, 2H), 7.35 (s, 1H), 7.45 (m, 1H), 7.75 (m, 1H), 7.80 (s, 1H), 8.25 (m, 1H).

### Beispiel 20

### 4-Amino-2-(4,4-dimethylimidazolin-2-on-1-yl)pyrimidin-5-carbonsäure-(Npentafluorpropyl-3-tritluormethyl-4-fluor-anilid)

Die Verbindung wird analog Beispiel 2, Verfahren B hergestellt. Ausbeute 5 %, Smp. 208°C; MS: m/e 545 (M⁺+1); 200 MHz ¹H-NMR (DMSO-d₆, ppm): 7.95-7.85 (s, 2H), 7.70-7.45 (m, 2H), 7.3 (s, 1 H), 7.0 (s, 2H), 4.78 (t, 2H), 3.58 (s, 2H), 4.78 (t, 2H), 3.58 (s, 2H), 1.20 (s, 6H)

### Beispiel 21

### 4-Amino-2-(4,4-dimethylimidazolidin-2-on-1-yl)pyrimidin-5-carbonsäure-(Nheptafluorpropyl-3-trifluormethyl-6-fluoranilid)

Die Verbindung wird analog Beispiel 2, Verfahren B hergestellt. Ausbeute 4 %; Smp. 200°C; MS: m/e 595 (M⁺+1); 200 MHz ¹H-NMR (DMSO-d₆, ppm): 8.25 (d, 1H), 7.82 (s, 1H), 7.82-7.70 (m,1H), 7.45 (t, 1H), 7.32 (s, 1H), 7.05 (s, 2H), 4.8 (t, 2H), 3.58 (s, 2H), 1.20 (s, 6H).

### Beispiel 22

### 4-Amino-2-(4,4-dimethyl-imidazolin-2-on-2-yl)-pyrimidin-5-carbonsäure-[N-(3-trifluormethyl-phenyl)-N-(2-fluor-ethyl)-amid

### 1.Stufe: Darstellung von 3-(1-Amidino-4,4-dimethyl-imidazolin-2-on)-2-cyanoacryl-säureethylester

2.11g (37 mmol) Kaliumhydroxid werden unter Erwärmen auf 70°C in 30 ml Isopropanol gelöst. Nach Abkühlen auf RT gibt man 8.9g (37 mmol) 1-Amidino-4,4-dimethyl-imidazolin-2-on zu und rührt noch eine Stunde nach. Es werden nun 6.34g (37 mmol) 2-Cyano-3-ethoxy-acrylsäureethylester, gelöst in 8ml Isopropanol, zugegeben. Die weiße Suspension wird dabei zeitweise dünnflüssiger, dann beginnt das Produkt auszufallen. Man rührt noch eine Stund bei 10°C nach, saugt ab und reinigt das so gewonnene Produkt durch Nachwaschen mit Isopropanol, Wasser, Isopropanol und MTB-Ether. Es wird anschließend im Vakuum bei 40°C bis zur Gewichtskonstanz getrocknet.
Ausbeute 8.9g (86% der Theorie); MS: m/e = 280.3 (M⁺ +1); 200 MHz ¹H-NMR (DMSO-d₆, ppm): 1.25(t, 3H), 1.30(s, 6H), 3.70(s, 2H), 4.15(q, 2H), 8.25(s, 1H), 8.60(s, 1H), 8.75(brs, 2H).

### 2. Stufe: Darstellung von 4-Amino-2-(4,4-dimethyl-imidazolin-2-on-1-yl)-pyrimidin-5-carbonsäureethylester

7.1g (25 mmol) des nach Stufe 1 hergestellten 3-(1-Amidino-4,4-dimethylimidazolin-2-on)-2-cyano-acryl-säureethylester werden in 30 ml Toluol suspendiert. Es wird mit 2.9g Trifluoressigsäure versetzt und auf 95°C erwärmt. Nach beendeter Cyclisierung wird auf RT abgekühlt und mit 30ml MTB-Ether versetzt. Das so gewonnene Rohprodukt wird säulenchromatographisch an Kiselgel gereinigt. Ausbeute 5.05g (72% der Theorie); MS: m/e = 280.2 (M⁺ +1); 200 MHz ¹H-NMR (DMSO-d₆, ppm): 1.25(s, 6H), 1.30(t, 3H), 3.70(s, 2H), 4.15(q, 2H), 7.40(s, 1H), 7.55(brs, 1H), 7.75(brs, 1H), 8.60(s, 1H).

### 3.Stufe: Darstellung von 4-Amino-2-(4,4-dimethyl-imidazolin-2-on-1-yl)-pyrimidin-5-carbonsäure

Zu einer Lösung von 0.8g Natriumhydroxid in 55ml Wasser gibt man 5.5g (19.7 mmol) des nach Stufe 2 hergestellten 4-Amino-2-(4,4-dimethyl-imidazolin-2-on-1-yl)-pyrimidin-5-carbonsäureethylesters. Die Suspension wird auf 70°C erwärmt und bis zum Verschwinden des Edukts (HPLC-Kontrolle) bei dieser Temperatur gerührt. Nun wird auf 50°C gekühlt und mit etwa 2ml 37% Hcl versetzt. Dabei fällt ein weißer Niederschlag aus. Es wird im Eisbad gekühlt und anschließend abgesaugt. Nachwaschen des Filterrückstandes mit Eiswasser und Trocknen im Vakuum liefert sauberes Produkt.
Ausbeute 3.30g (66% der Theorie); MS: m/e = 280.2 (M⁺ +1); 200 MHz ¹H-NMR (DMSO-d₆, ppm): 1.25(s, 6H), 1.30(t, 3H), 3.70(s, 2H), 4.15(q, 2H), 7.40(s, 1H), 7.55(brs,1H), 7.75(brs, 1H), 8.60(s, 1H).

Die in der Beschreibung verwendeten Abkürzungen haben folgende Bedeutungen:
- DME: Dimethoxyethan
- NEt₃: Triethylamin
- TMSCI: Trimethylchlorsilan
- LDL: low-density-lipoprotein
- h: Stunde
- NMP: N-Methyl-pyrrolidon
- DMF: Dimethylformamid
- TOTU: o-[(Cyano-(ethoxycarbonyl)-methyliden)-amino-1,1,3,3-tetramethyl]-uronium-tetrafluoroborat

## Patentansprüche

1. Tertiäre 4-Amino-2-ureido-pyrimidin-5-carbonsäure-amide der Formel I, 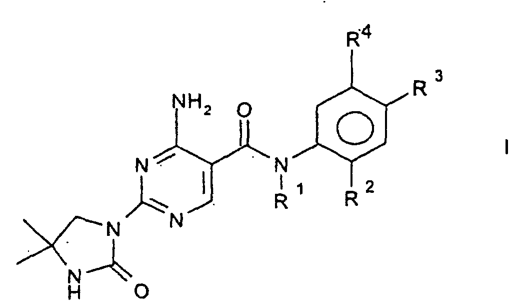 worin bedeuten
R¹ 2,2,2-Trifluorethyl, 2,2,3,3,3-Pentafluorpropyl, 2,2,3,3,4,4,4-Heptafluorbutyl,
R² Fluor, Chlor, Wasserstoff, -CF₃, -OCF₃,
R³ Fluor, Chlor, Wasserstoff, -CF₃, -OCF₃,
R⁴ CF₃,
sowie deren physiologisch verträgliche Säureadditionssalze.

2. Verfahren zur Herstellung von Verbindungen der Formel I, gemäß Anspruch 1, **dadurch gekennzeichnet, dass** man nach folgendem Formelschema 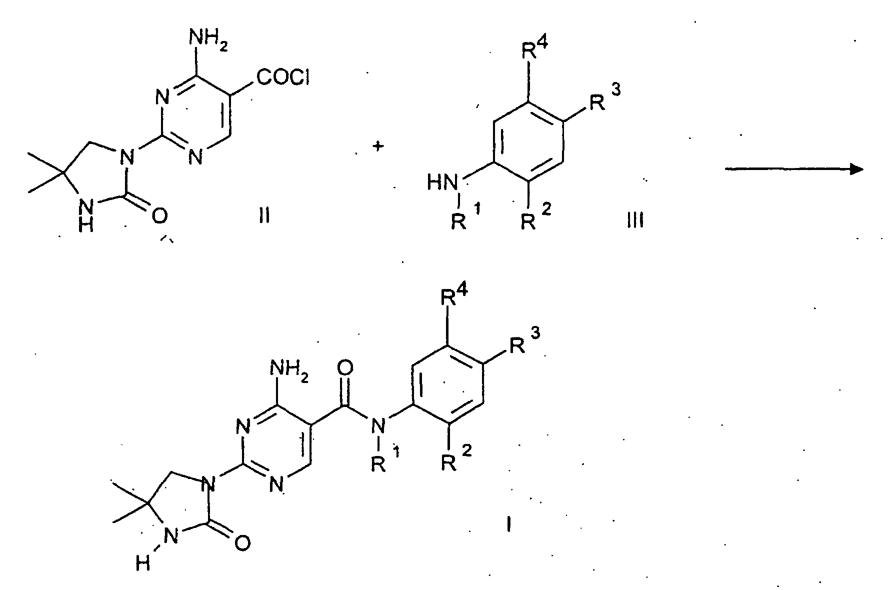 eine Verbindung der Formel II mit einer Verbindung der Formel III, in der R¹, R², R³ und R⁴ die zu Formel I angegebenen Bedeutungen haben, bei einer Temperatur von 0 °C bis 200 °C in einem geeigneten Lösungsmittel zu einer Verbindung der Formel I umsetzt und die erhaltene Verbindung der Formel I gegebenenfalls in ein physiologisch verträgliches Salz überführt oder ein erhaltenes Salz in ein physiologisch verträgliches Salz überführt.

3. Verfahren zur Herstellung von Verbindungen der Formel I gemäß Anspruch 1, **dadurch gekennzeichnet, dass** man nach folgendem Formelschema 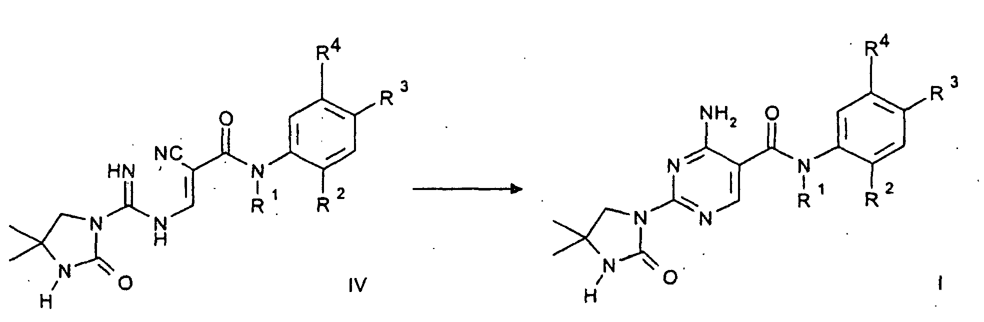 eine Verbindung der Formel IV, in der R¹, R², R³ und R⁴ die zu Formel I angegebenen Bedeutungen haben, zu einer Verbindung der Formel I cyclisiert.

4. Verfahren zur Herstellung von Verbindungen der Formel gemäß Anspruch 1, **dadurch gekennzeichnet, dass** man nach folgendem Formelschema 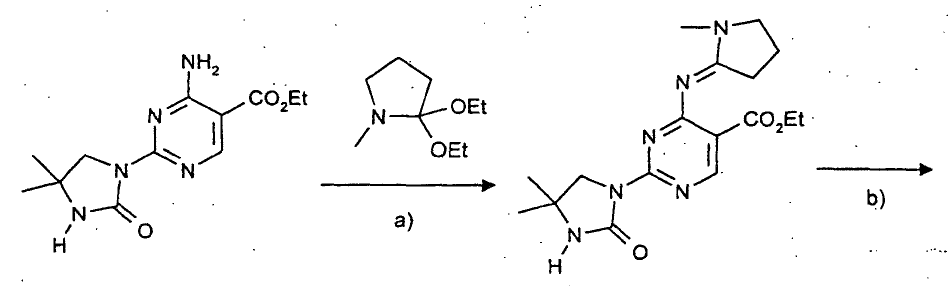 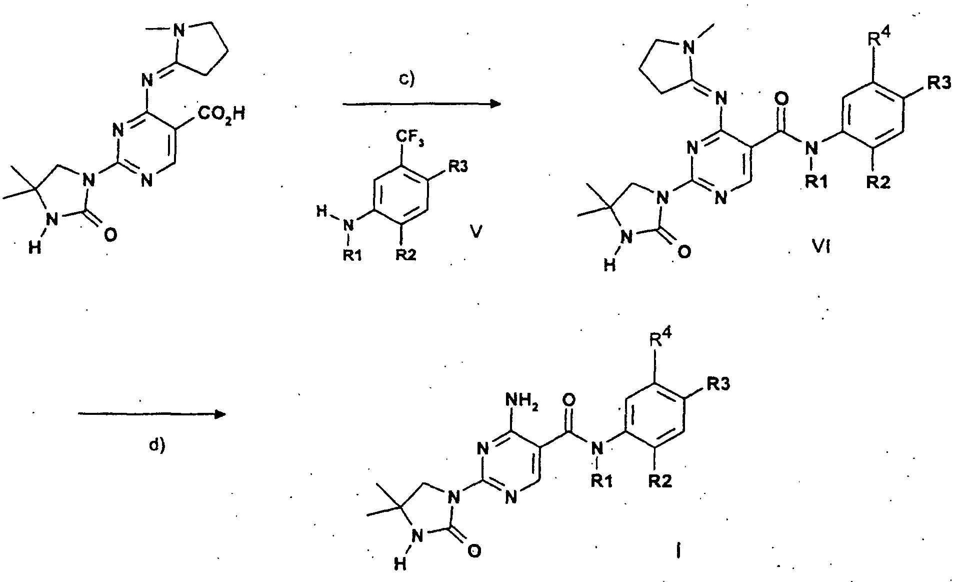
a) 4-Amino-2-(4,4-dimethyl-imidazolin-2-on-1-yl)-pyrimidin-carbonsäureethylester mit 2,2-Diethoxy-1-methyl-pyrrolidin in einem geeigneten Lösungsmittel, bei einer Temperatur von 0 ° bis 150 °C zu 4-(1-Methyl-pyrrolidin-2-ylidenamino)-2-(4,4-dimethyl-imidazolin-2-on-1-yl)-pyrimidin-5-carbonsäureethylester umsetzt,
b) den erhaltenen 4-(1-Methyl-pyrrolidin-2-ylidenamino)-2-(4,4-dimethyl-imidazolin-2-un-1-yl)-pyrimidin-5-carbönsäureethylester mit NaJ und TMSCI in einem geeigneten Lösungsmittel bei einer Temperatur von 0 ° bis 150 °C zu 4-(1-Methylpyrrolidin-2-ylidenamino)-2-(4,4-dimethyl-imidazolin-2-on-1-yl)pyrimidin-5-carbonsäure umgesetzt,
c) die erhaltene 4-(1-Methyl-pyrrolidin-2-ylidenamino)-2-(4,4-dimethyl-imidazolin-2-on-1-yl)-pyrimidin-5-carbonsäure in einem geeigneten Lösungsmittel bei einer Temperatur von 0 ° bis 150 °C in Gegenwart von TOTU und einer Hilfsbase mit einer Verbindung der Formel V umgesetzt, in der R¹, R², R³ und R⁴ die zu Formel I angegebenen Bedeutungen haben und
d) die erhaltene Verbindung der Formel VI, in der R¹, R², R³ und R⁴ die zu Formel I angegebenen Bedeutungen haben, in einem geeigneten Lösungsmittel, bei einer Temperatur von 0 ° - 150 °C in Gegenwart einer Hilfsbase, wie z. B. wäßrige Ammoniaklösung mit Ethylendiamin zu einer Verbindung der Formel I umgesetzt.

5. Arzneimittel enthaltend eine oder mehrere Verbindung(en) gemäß Anspruch 1.

6. Verbindungen gemäß Anspruch 1 zur Behandlung von Lipidstoffwechselstörungen.

7. Verbindungen gemäß Anspruch 1 zur Behandlung von Hyperlipidämie.

## Claims

1. A tertiary 4-amino-2-ureidopyrimidine-5-carboxamide of the formula I 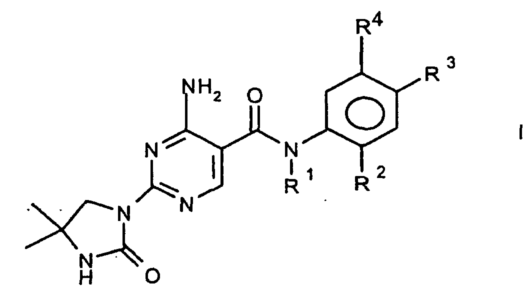 in which
R¹ is 2,2,2-trifluoroethyl, 2,2,3,3,3-pentafluoropropyl, 2,2,3,3,4,4,4-heptafluorobutyl,
R² is fluorine, chlorine, hydrogen, -CF₃, -OCF₃,
R³ is fluorine, chlorine, hydrogen, -CF₃, -OCF₃,
R⁴ is CF₃,
or its physiologically tolerable acid addition salts.

2. A process for the preparation of compounds of the formula I, as claimed in claim 1, which comprises, according to the following reaction scheme 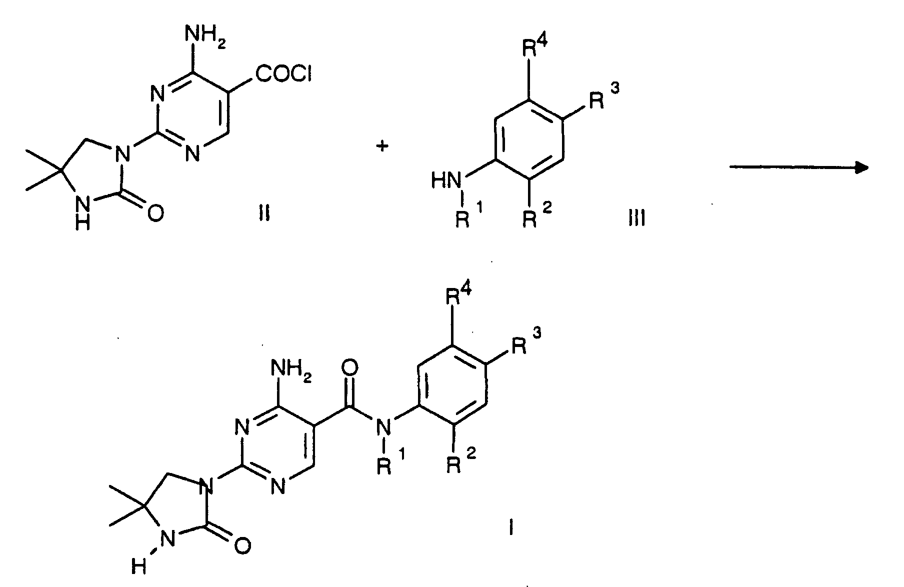 reacting a compound of the formula II with a compound of the formula III, in which R¹, R², R³ and R⁴ have the meanings indicated for formula I, at a temperature from 0°C to 200°C in a suitable solvent to give a compound of the formula I and optionally converting the compound of the formula I obtained into a physiologically tolerable salt or converting a salt obtained into a physiologically tolerable salt.

3. A process for the preparation of compounds of the formula I as claimed in claim 1, which comprises, according to the following reaction scheme 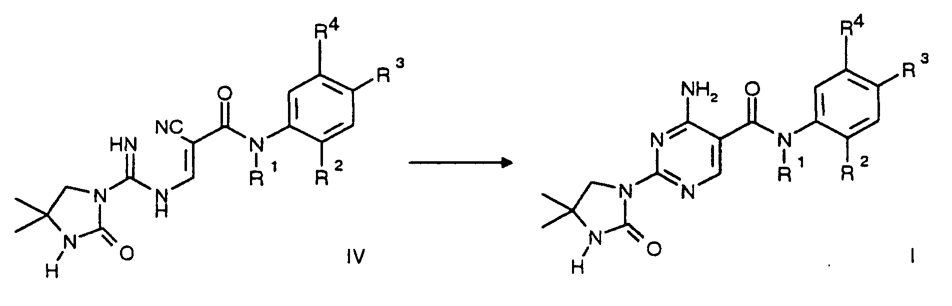 cyclizing a compound of the formula IV, in which R¹, R², R³ and R⁴ have the meanings indicated for formula I, to a compound of the formula I.

4. A process for the preparation of compounds of the formula I as claimed in claim 1, which comprises, according to the following reaction scheme 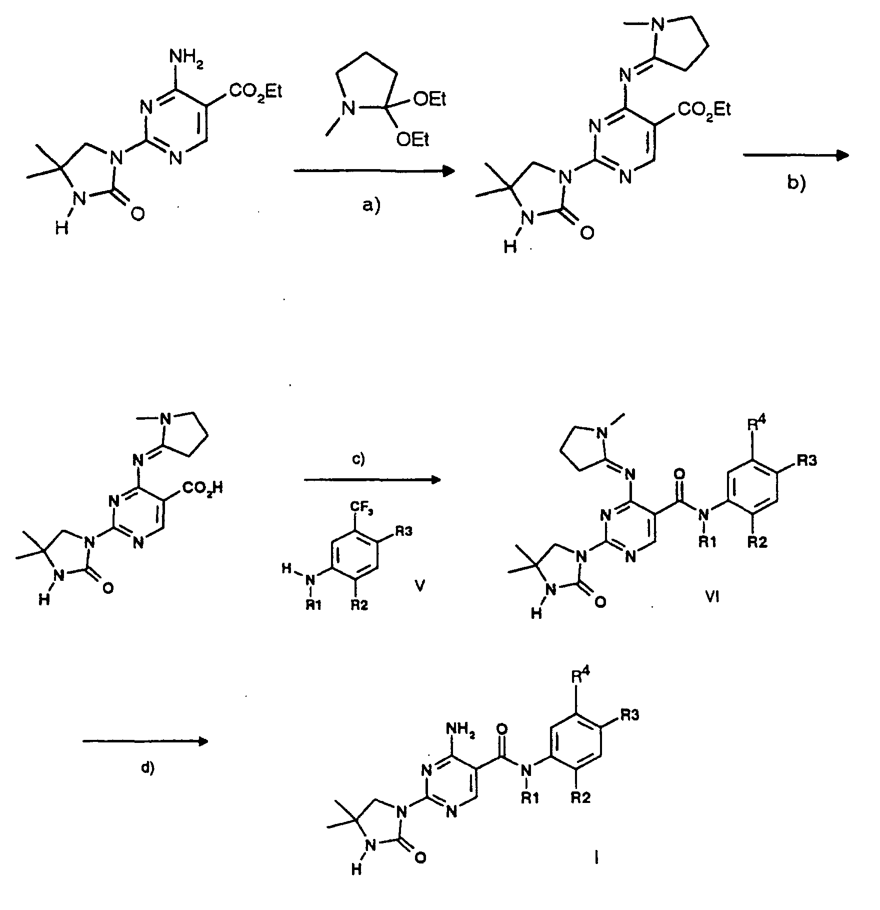
a) reacting ethyl 4-amino-2-(4,4-dimethylimidazolidin-2-on-1-yl)-pyrimidinecarboxylate with 2,2-diethoxy-1-methylpyrralidine in a suitable solvent, at a temperature from 0° to 150°C, to give ethyl 4-(1-methylpyrrolidin-2-ylideneamino)-2-(4,4-dimethylimidazolidin-2-on-1-yl)-pyrimidine-5-carboxylate,
b) reacting the ethyl 4-(1-methylpyrrolidin-2-ylideneamino)-2-(4,4-dimethylimidazolidin-2-on-1-yl)pyrimidine-5-carboxylate obtained with Nal and TMSCI in a suitable solvent at a temperature from 0° to 150°C to give 4-(1-methylpyrrolidin-2-ylideneamino)-2-(4,4-dimethylimidazolidin-2-on-1-yl)-pyrimidine-5-carboxylic acid,
c) reacting the 4-(1-methylpyrrolidin-2-ylideneamino)-2-(4,4-dimethyl-imidazolidin-2-on-1-yl)-pyrimidine-5-carboxylic acid obtained in a suitable solvent at a temperature from 0° to 150°C in the presence of TOTU and of an auxiliary base with a compound of the formula V, in which R¹, R², R³ and R⁴ have the meanings indicated for formula I and
d) reacting the compound of the formula VI obtained, in which R¹, R², R³ and R⁴ have the meanings indicated for formula I, in a suitable solvent, at a temperature of 0° - 150°C in the presence of an auxiliary base, such as, for example, aqueous ammonia solution, with ethylenediamine to give a compound of the formula I.

5. A pharmaceutical comprising one or more compounds as claimed in claim 1.

6. A compound as claimed in claim 1 for the treatment of disorders of lipid metabolism.

7. A compound as claimed in claim 1 for the treatment of hyperlipidemia.

## Revendications

1. Amides tertiaires de l'acide 4-amino-2-uréido-pyrimidine-5-carboxylique de formule I, 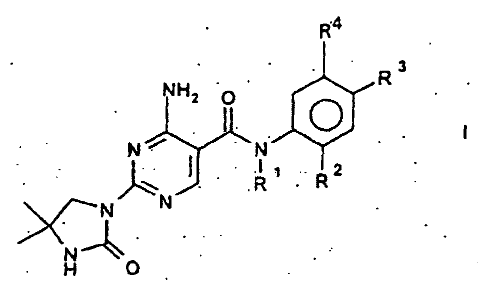 dans laquelle
R¹ signifie 2,2,2-trifluoroéthyle, 2,2,3,3,3-pentafluoropropyle, 2,2,3,3,4,4,4-heptafluorobutyle,
R² signifie fluor, chlore, hydrogène, -CF₃, -OCF₃,
R³ signifie fluor, chlore, hydrogène, -CF₃, -OCF₃,
R⁴ signifie CF₃,
ainsi que leurs sels d'addition d'acide physiologiquement acceptables.

2. Procédé pour la préparation de composés de formule I, selon la revendication 1, **caractérisé en ce qu'**on transforme, selon le schéma de formules suivant, 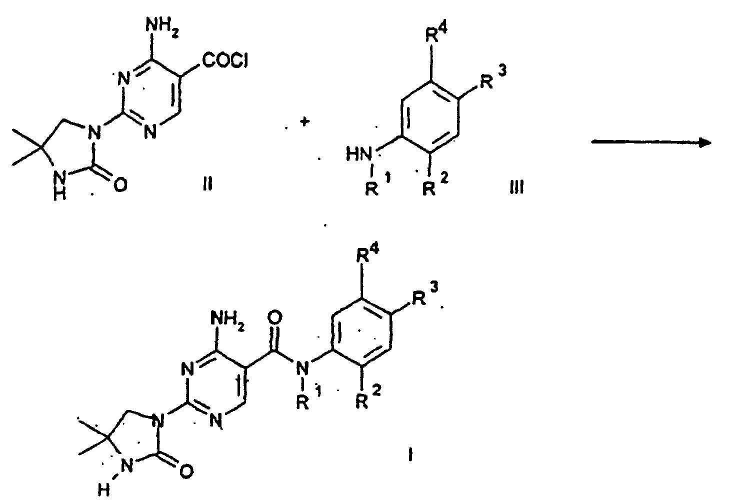 un composé de formule II avec un composé de formule III, dans laquelle R¹, R², R³ et R⁴ ont les significations indiquées pour la formule I, à une température de 0°C à 200°C, dans un solvant approprié, en un composé de formule I et on transforme le composé de formule I obtenu le cas échéant en un sel physiologiquement acceptable ou on transforme un sel obtenu en un sel physiologiquement acceptable.

3. Procédé pour la préparation de composés de formule I, selon la revendication 1, **caractérisé en ce qu'**on cyclise, selon le schéma de formules suivant 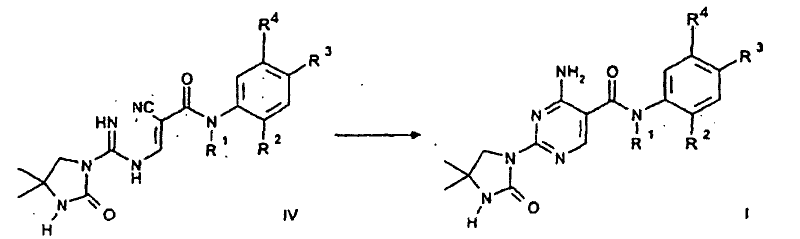 un composé de formule IV, dans lequel R¹, R², R³ et R⁴ ont la signification indiquée pour la formule I, en un composé de formule I.

4. Procédé pour la préparation de composés de formule I, selon la revendication 1, **caractérisé en ce qu'**on transforme, selon le schéma de formules suivant 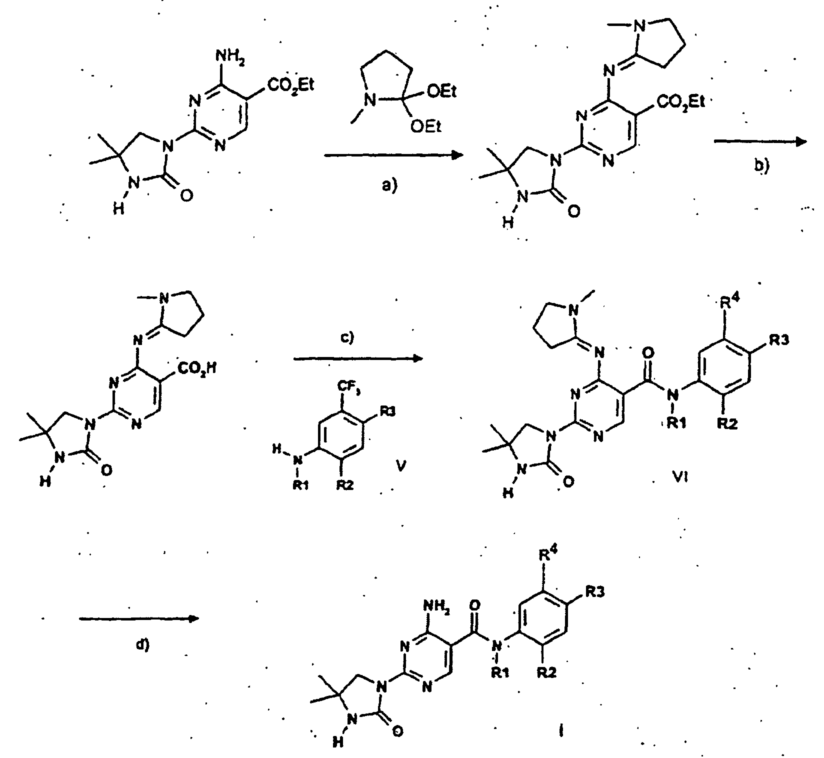
a) l'ester éthylique de l'acide 4-amino-2-(4,4-diméthylimidazolidin-2-on-1-yl)pyrimidine-carboxylique avec de la 2,2-diéthoxy-1-méthylpyrrolidine dans un solvant approprié à une température de 0° à 150°C en ester éthylique de l'acide 4-(1-méthylpyrrolidin-2-ylidèneamino)-2-(4,4-diméthylimidazolidin-2-on-1-yl)pyrimidine-5-carboxylique,
b) l'ester éthylique de l'acide 4-(1-méthylpyrrolidin-2-ylidèneamino)-2-(4,4-diméthylimidazolidin-2-on-1-yl)pyrimidine-5-carboxylique obtenu avec du NaI et du TMSCl dans un solvant approprié à une température de 0°C à 150°C en acide 4-(1-méthylpyrrolidin-2-yl-idèneamino)-2-(4,4-diméthyl-imidazolidin-2-on-1-yl)pyrimidine-5-carboxylique,
c) l'acide 4-(1-méthylpyrrolidin-2-ylidène-amino)-2-(4,4-diméthylimidazolidin-2-on-1-yl)pyrimidine-5-carboxylique obtenu dans un solvant approprié à une température de 0°C à 150°C en présence de TOTU et d'une base auxiliaire avec un composé de formule V, dans laquelle R¹, R², R³ et R⁴ ont les significations indiquées dans la Formule I et
d) le composé de formule VI obtenu, dans laquelle R¹, R² R³ et R⁴ ont les significations indiquées dans la Formule I, dans un solvant approprié, à une température de 0°C à 150°C, en présence d'une base auxiliaire, telle que par exemple une solution aqueuse d'ammoniaque, avec de l'éthylènediamine en un composé de formule I.

5. Médicament, contenant un ou plusieurs composés selon la revendication 1.

6. Composés selon la revendication 1 pour le traitement de troubles du métabolisme des lipides.

7. Composés selon la revendication 1 pour le traitement de l'hyperlipidémie.
